# EUROPEAN PATENT APPLICATION

(11) **EP 4 461 331 A1**
(43) Date of publication of application: **13.11.2024**
(21) Application number: 22919012.9
(22) Date of filing: 24.11.2022
(51) Int. Cl.: A61M 5/168, A61M 5/142

(54) **LIQUID MEDICINE INJECTION DEVICE FOR SENSING LEAKAGE**

(30) Priority: 04.01.2022 KR 20220001046; 20.04.2022 KR 20220048653
(71) Applicant: Eoflow Co., Ltd., Seongnam-si, Gyeonggi-do 13605 (KR)
(72) Inventor: KIM, Jesse Jaejin, Seongnam-si Gyeonggi-do 13562 (KR); MIN, Kyoung In, Gimpo-si Gyeonggi-do 10129 (KR); JU, Sang A, Anyang-si Gyeonggi-do 13903 (KR)
(74) Representative: Cabinet Netter
(86) International application number: PCT/KR2022/018702
(87) International publication number: WO 2023/132475

(57) **Abstract**

Embodiments of the present invention relate to a liquid medicine injection device that is attached to a user's body to inject a liquid medicine. In order to sense liquid leakage, the liquid medicine injection device includes: a leakage sensing unit mounted on a circuit board in the liquid injection device and comprising at least one leakage sensing pad for generating a leakage signal when sensing a liquid; and a processor mounted on the circuit board and electrically connected to the leakage sensing unit so as to receive the leakage signal and to determine whether a leakage has occurred, thereby notifying a user of information related to the leakage when the leakage has occurred.

## Description

### TECHNICAL FIELD

Embodiments of the present disclosure relate to a liquid medicine injection device that senses a leakage of liquid or introduction of external liquid.

### BACKGROUND ART

Typically, a liquid medicine injection device such as an insulin injection device is used to inject a liquid medicine into a patient's body. Such liquid medicine injection devices are used by professional medical staffs such as doctors and nurses, but in most cases, they are used by the general public, such as patients themselves or their guardians.

Diabetic patients, especially, juvenile diabetic patients need to inject medicinal fluid, such as insulin, into their bodies at set intervals. A patch type liquid medicine injection device that is attached to the human body for a certain period of time is being developed. This type liquid medicine injection device may be used while being attached to the patient's body, such as the abdomen or waist, in the form of a patch for a certain period of time.

In the case of a liquid medicine injection device attached to the human body, a medicinal fluid inside the liquid medicine injection device may leak due to excessive movement of the patient, shocks, or unexpected circumstances. There is also a case where external liquid leaks into the liquid medicine injection device when the device is exposed to the liquid at a temperature or pressure or for a time exceeding waterproofing.

In order to increase effects through liquid medicine injection, a liquid medicine injection device must be controlled to precisely inject a set amount of liquid medicine into a patient's body at a set time. However, if the patient or user fails to sense leakage of the liquid medicine in advance and deals with it late, a problem may arise in terms of controlling an amount of liquid medicine to be injected. In addition, there is a problem that leaked liquid medicine or external liquid infiltrates into a circuit board within the liquid medicine injection device. This causes the liquid medicine injection device to be turned off without the patient's intention or to be damaged.

### DESCRIPTION OF EMBODIMENTS

### TECHNICAL PROBLEM

Embodiments of the present disclosure provide a liquid medicine injection device that senses a leakage of a liquid medicine or an introduction of an external liquid and notifies a user of a result of the sensing. Technical problems to be solved in embodiments of the present disclosure are not limited to the problem to be solved, and other technical problems to be solved can be inferred from the following embodiments.

### SOLUTION TO PROBLEM

According to an aspect of the present disclosure, there is provided a liquid medicine injection device that is attached to a user's body to inject a liquid medicine, the device including: a leakage sensing unit that is mounted on a control module and including at least one leakage sensing pad for generating a leakage signal when sensing a liquid; and a processor that is mounted on the control module and electrically connected to the leakage sensing unit, to determine whether a leakage has occurred by receiving the leakage signal and notify the user of information related to the leakage when the leakage has occurred.

Here, the liquid medicine injection device may further include: an alarm unit that is electrically connected to the processor to notify the user of the information related to the leakage by generating an alarm of sound, light, or vibration; and a remote device that notifies the user of the information related to the leakage through communication with the processor.

Here, the information related to the leakage may include at least one of whether the leakage has occurred, a location where the leakage has occurred, and a type of leakage that has occurred.

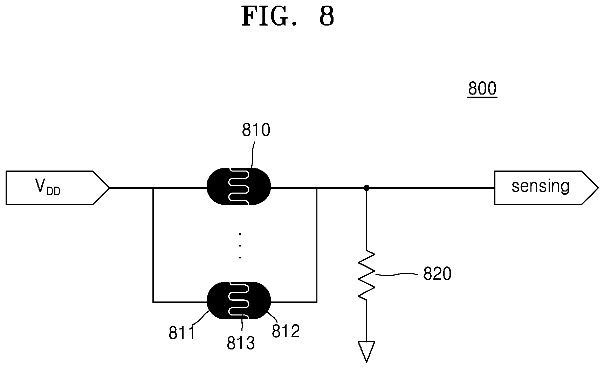

Here, referring to an embodiment of FIG. 8, the leakage sensing pad may include a first pattern electrically connected to a power source, and a second pattern electrically connected to the processor and spaced a certain gap from the first pattern, and the leakage sensing unit may further include: the at least one leakage sensing pad connected in parallel; and a voltage divider interposed between the at least one leakage sensing pad connected in parallel and the processor.

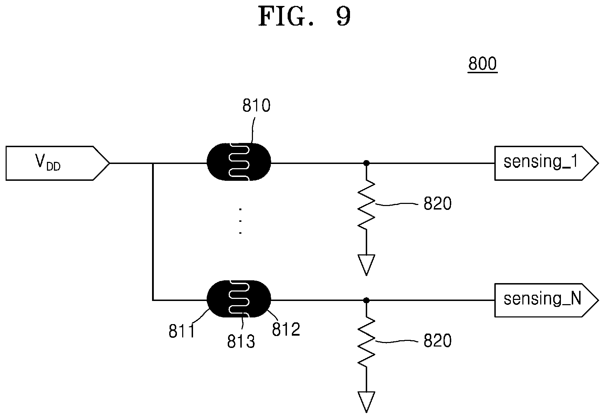

Here, referring to an embodiment of FIG. 9, the leakage sensing unit may include: the at least one leakage sensing pad each having a first pattern electrically connected to a power source, and a second pattern electrically connected to the processor and spaced a certain gap from the first pattern; and at least one voltage divider interposed between the second pattern of the at least one leakage sensing pad and the processor, the at least one leakage sensing pad may each be mounted on the control module at a different position, and the leakage sensing unit may generate a leakage signal from the at least one leakage sensing pad and input the generated leakage signal to the processor.

Here, referring to the embodiment of FIG. 8 or FIG. 9, the leakage signal may be a voltage value, and the processor may determine a type of leakage based on the voltage value and notify the user of the determined type of leakage as the information related to the leakage.

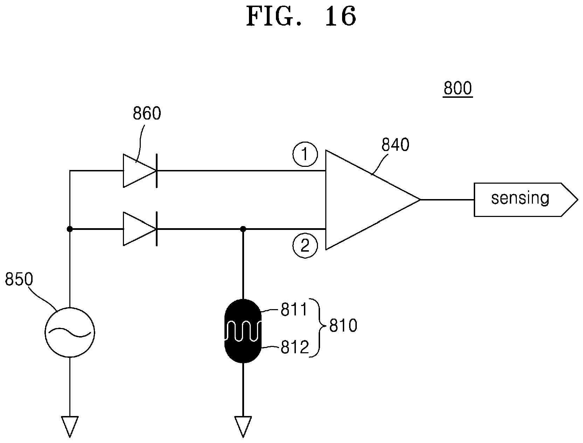

Here, referring to an embodiment of FIG. 16, the leakage sensing unit may include a comparator that receives a reference signal through a first input part, receives a transformed signal from a second input part, and outputs the leakage signal, and the leakage sensing pad may be electrically connected to the second input part, to input the transformed signal to the second input part when the leakage has occurred.

Here, referring to the embodiment of FIG. 16, the leakage signal may have a waveform, and the processor may determine a type of leakage based on at least one of a duty, pulse width, and delay of the waveform, and notify the user of the determined type of leakage as the information related to the leakage.

Here, the liquid medicine injection device may further include: a needle assembly that is mounted on a first receiving portion formed in the control module and has a needle; and a reservoir unit that is mounted on a second receiving portion formed in the control module, accommodates the liquid medicine, and is connected to one end of the needle to deliver the accommodated liquid medicine to the user.

Here, the at least one leakage sensing pad may be located on the control module to be adjacent to the reservoir unit and to the needle assembly.

Other aspects, features, and advantages in addition to those described above will become apparent from the following drawings, claims, and detailed description of the invention.

These general and specific aspects may be practiced using any device, system, method, computer program, or combination of any device, system, method, or computer program.

### EFFECTS OF THE INVENTION

According to embodiments of the present disclosure, when a leakage has occurred in a liquid medicine injection device, the leakage can be sensed and recognized by a user, which provides effects of suppressing in advance damage to a circuit board and forced turn-off of a system due to the leakage and enhancing reliability of the device.

According to embodiments of the present disclosure, when a leakage has occurred in a liquid medicine injection device, it is possible to determine not only whether the leakage has occurred, but also a location where a liquid has been leaked or introduced or a type of liquid that has been leaked or introduced. This allows a user to respond to such a leakage issue quickly and accurately.

The scope of the present disclosure is not limited by these effects.

### BRIEF DESCRIPTION OF DRAWINGS

FIG. 1 is a diagram of a liquid medicine injection system according to one exemplary embodiment of the present disclosure.
FIG. 2 is an exploded perspective view illustrating an internal disposition of the liquid medicine injection device of FIG. 1.
FIG. 3 is a bottom perspective view of the liquid medicine injection device of FIG. 2.
FIG. 4 is a diagram illustrating a state in which an attachment part has been removed from FIG. 3.
FIG. 5 is a diagram showing a bottom surface of a control module, in a state where a bottom cover has been removed from FIG. 4.
FIG. 6 is a plan view illustrating a top surface of the control module in a state where several components have been removed from FIG. 2, and a partial enlarged view illustrating the inside of a reservoir unit.
FIG. 7 is a block diagram functionally illustrating a configuration of a control module according to one exemplary embodiment of the present disclosure.
FIG. 8 is a diagram illustrating a leakage sensing unit according to one exemplary embodiment of the present disclosure.
FIG. 9 is a diagram illustrating a leakage sensing unit according to another exemplary embodiment of the present disclosure.
FIGS. 10 to 13 are diagrams illustrating various types of leakage sensing pads.
FIG. 14 is a schematic view illustrating positions of leakage sensing pads on a control module.
FIG. 15 is a flowchart for a leakage management method of a liquid medicine injection system.
FIG. 16 is a diagram illustrating a leakage sensing unit according to still another exemplary embodiment of the present disclosure.
FIGS. 17 and 18 are views illustrating signals related to the leakage sensing unit of FIG. 16.

### BEST MODE

According to one embodiment of the present disclosure, there is provided a liquid medicine injection device that is attached to a user's body to inject a liquid medicine, which includes a leakage sensing unit mounted on a control module and having at least one leakage sensing pad for generating a leakage signal when liquid is sensed; and a processor mounted on the control module and electrically connected to the leakage sensing unit so as to receive the leakage signal and to determine whether a leakage has occurred, thereby notifying a user of information related to the leakage when the leakage has occurred.

### MODE OF DISCLOSURE

Since the present disclosure can be modified in various ways and can have various embodiments, specific embodiments will be illustrated in the drawings and specifically described in the detailed description. The effects and features of the present disclosure and methods for achieving the same will be clearly understood by referring to embodiments to be described in detail below along with the drawings. However, the present disclosure is not limited to the embodiments disclosed below and may be implemented in various forms.

Description will now be given in detail of embodiments disclosed herein, with reference to the accompanying drawings. For description with reference to the drawings, the same or equivalent components may be provided with the same reference numerals, and a redundant description thereof will be omitted.

In embodiments disclosed below, a singular representation may include a plural representation unless it represents a definitely different meaning from the context.

In embodiments disclosed below, terms such as "include" or "has" should be understood that they are intended to indicate an existence of features or components, disclosed in this specification, and also it is not excluded in advance that one or more features or components are likewise utilized.

If an embodiment can be implemented differently, the order of specific steps may be performed differently from the order described. For example, two steps described in succession may be performed substantially at the same time or may be performed in a reverse order opposite to that described.

In the drawings, the sizes of components may be enlarged or exaggerated or reduced for convenience of explanation. For example, a size and a thickness of each component illustrated in the drawings are illustrative for convenience of description, and the embodiment below is not limited to the size and the thickness of the component illustrated.

The expression "liquid leakage or leakage" used throughout this specification not only refers to a liquid that leaks out of a specific container or the liquid that has leaked, but also refers to a liquid that is introduced into a specific space from outside or the liquid that has been introduced.

The expression "leakage sensing" used throughout this specification may be a concept encompassing a process of sensing, detecting, or analyzing later both leaked liquid and introduced liquid, as described above.

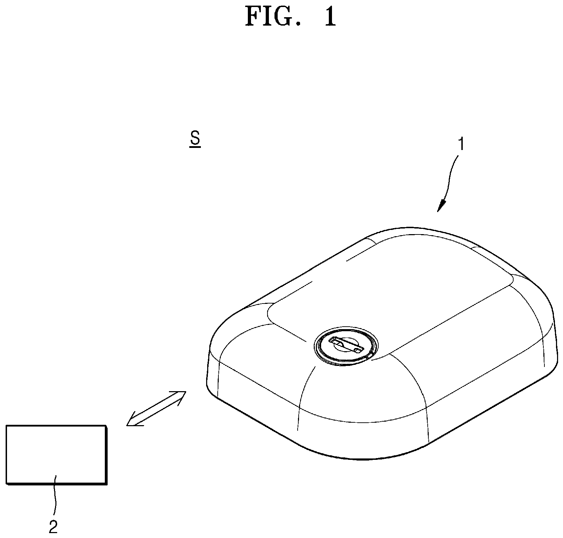

FIG. 1 is a diagram illustrating a liquid medicine injection system S according to one exemplary embodiment of the present disclosure.

Referring to FIG. 1, the liquid medicine injection system S includes a liquid medicine injection device 1 and a remote device 2.

The liquid medicine injection device 1 may be attached to a target (object) into which a liquid medicine is to be injected, and inject the liquid medicine, which is stored therein, by a set dosage into a user. In an optional embodiment, the liquid medicine injection device 1 may be mounted on the user's body. Additionally, in another optional embodiment, the liquid medicine injection device 1 may also be mounted on an animal to inject a liquid medicine.

The liquid medicine injection device 1 may be used for various purposes depending on the type of liquid medicine to be injected. For example, liquid medicine may include insulin-based liquid medicine for diabetic patients, liquid medicine for the pancreas, liquid medicine for the heart, and other various types of liquid medicine.

The liquid medicine injection device 1 may be connected to the remote device 2 that is wiredly or wirelessly connected. The user may use the liquid medicine injection device 1 by manipulating the remote device 2 and monitor the usage status of the liquid medicine injection device 1. For example, the user may monitor, through the remote device 2, a dosage of liquid medicine injected from the liquid medicine injection device 1, the number of times the liquid medicine is injected, an amount of liquid medicine stored in a reservoir, user's bio information, etc., and activate the liquid medicine injection device 1 based on results of the monitoring. In addition, the user may monitor whether a leakage of liquid medicine from the liquid medicine injection device 1 or infiltration of external liquid has occurred through the remote device 2, and effectively manage the liquid medicine injection device 1 based on a result of the monitoring.

In one embodiment, the remote device 2 refers to a communication terminal that can use an application in a wired or wireless communication environment. Here, the remote device 2 may be the user's portable terminal. To explain this in more detail, the remote device 2 may be any type of a computer (e.g., desktop, laptop, tablet, etc.), a media computing platform (e.g., cable, satellite settop box, digital video recorder), a handheld computing device (e.g., PDA, email client, etc.), any type of cellular phone, any type of wearable device that can be used by being attached or mounted on the user's body, or any other type of computing or communication platform, but the present disclosure is not limited thereto.

The liquid medicine injection device 1 and the remote device 2 may communicate with each other through a communication network. At this time, the communication network refers to a communication network that provides an access path through which the remote device 2 can transmit and receive data after accessing a service server (not shown). Communication networks include, for example, wired networks such as local area networks (LANs), wide area networks (WANs), metropolitan area networks (MANs), and integrated service digital networks (ISDNs), and the like, or wireless networks such as wireless LANs, CDMA, Bluetooth, and satellite communications, and the like, but the present disclosure is not limited thereto.

Referring to FIG. 1, the remote device 2 is shown as a single device, but the present disclosure is not necessarily limited thereto and may include a plurality of devices capable of communicating with the liquid medicine injection device 1.

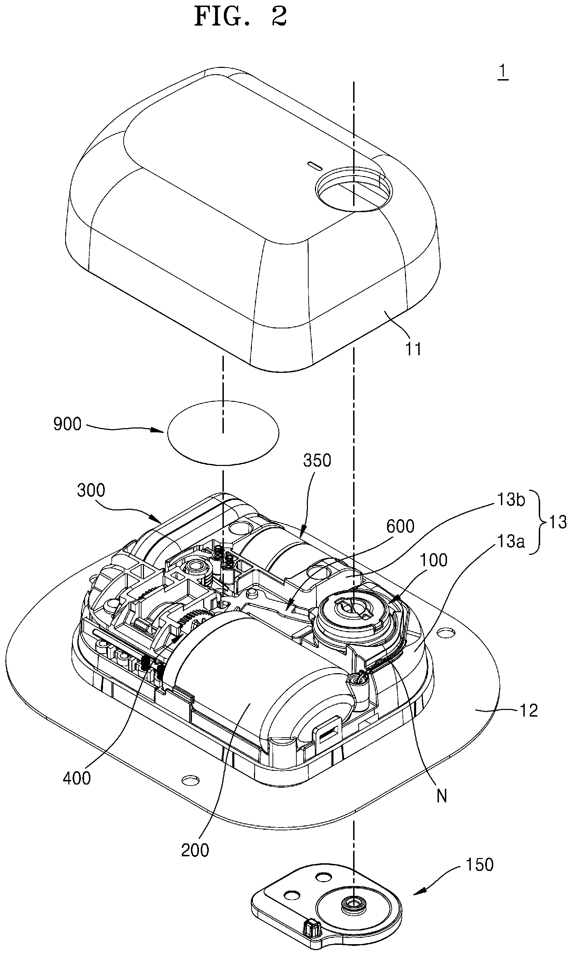

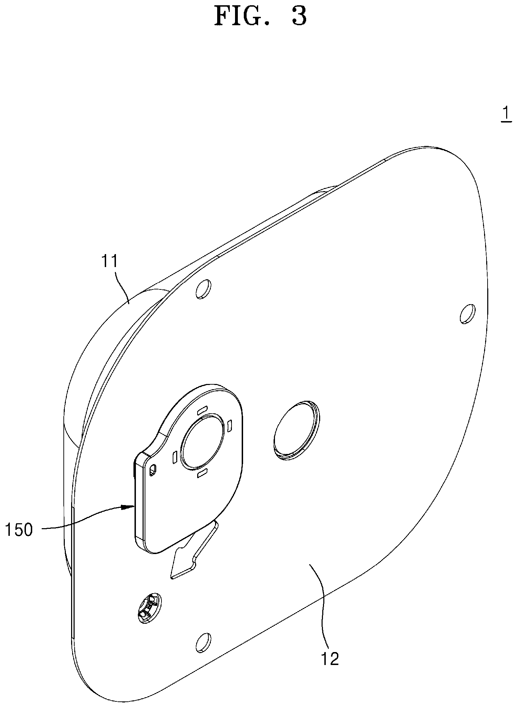

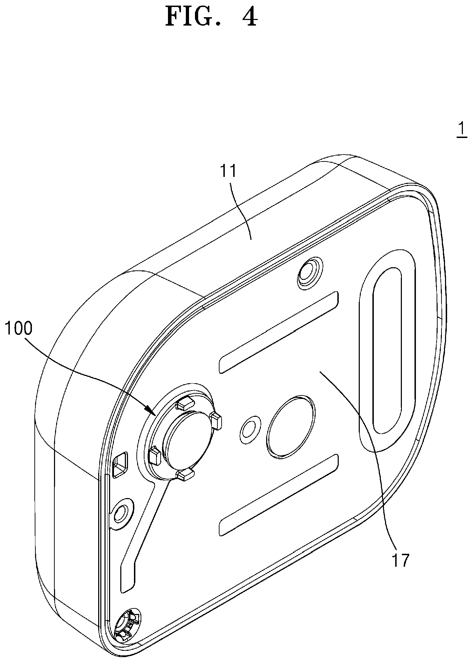

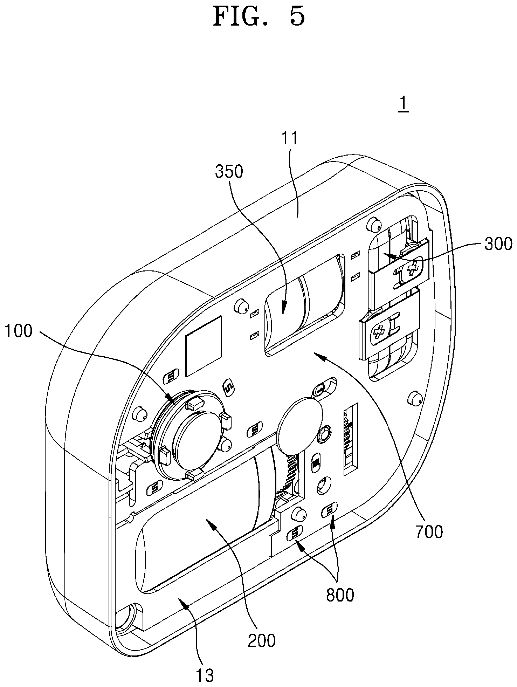

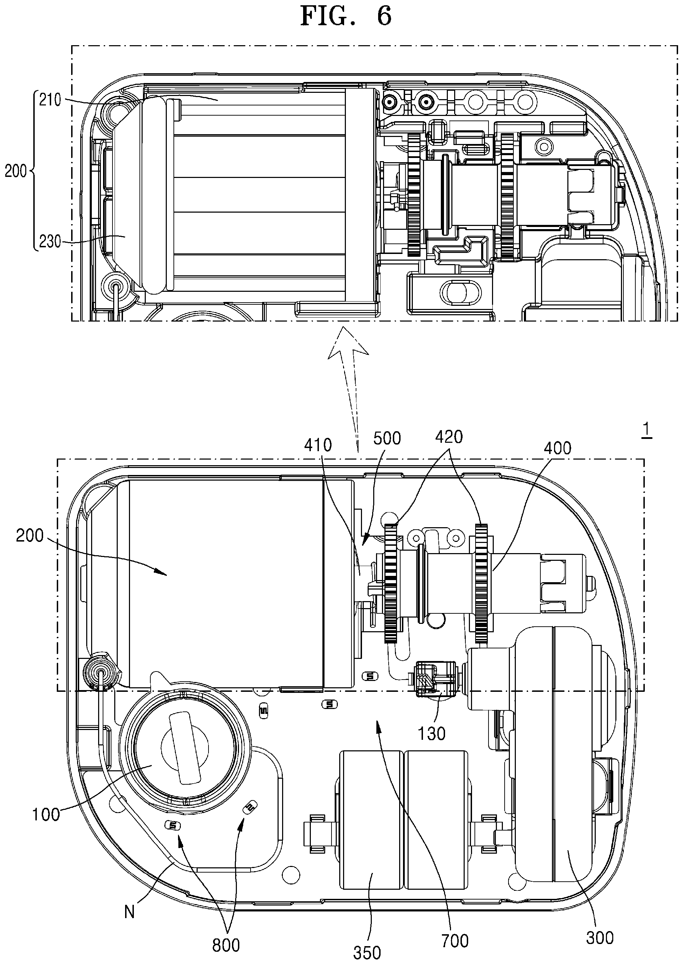

FIG. 2 is an exploded perspective view illustrating an internal disposition of the liquid medicine injection device 1 of FIG. 1. FIG. 3 is a bottom perspective view of the liquid medicine injection device 1. FIG. 4 is a diagram illustrating a state in which an attachment part 12 has been removed from FIG. 3. FIG. 5 is a diagram illustrating a bottom surface of a control module 700, in a state where a bottom cover 17 has been removed from FIG. 4. FIG. 6 is a plan view illustrating a top surface of the control module 700, and also a partial enlarged view illustrating the inside of a reservoir unit 200. Hereinafter, a description will be given with reference to FIGS. 2 to 6.

Referring to FIG. 2, one embodiment of the liquid medicine injection device 1 may include an external housing 11 covering the outside, and an attachment part 12 located adjacent to a user's skin. The liquid medicine injection device 1 includes a plurality of components disposed in an inner space between the external housing 11 and the attachment part 12. A separate adhesion means may be further interposed between the attachment part 12 and the user's skin, and the liquid medicine injection device 1 may be fixed to the skin by the adhesion means.

The liquid medicine injection device 1 may include a base body 13, a needle assembly 100, a reservoir unit 200, a pump module 300, a drive unit 400, a clutch unit 500, a trigger member 600, a control module 700, a leakage sensing unit 800, a battery 350, an alarm unit 900, and a needle cover assembly 150.

The base body 13 forms the basic frame of the external housing 11 and is mounted in the inner space of the external housing 11. The base body 13 may be provided in plurality. In one embodiment, the plurality of bodies may include a first body 13a that covers a top of internal components, and a second body 13b that covers a bottom of the internal components. The first body 13a and the second body 13b may be assembled to each other, such that the internal components of the liquid medicine injection device 1 are fixed at preset positions. In another embodiment, the base body 13 may be formed as an integrated frame.

The needle assembly 100 may be mounted to a receiving portion implemented in the base body 13 and the control module 700. The needle assembly 100 may include a needle N. One end of the needle N may be connected to the reservoir unit 200 to deliver a liquid medicine, and another end may be inserted into the user so that the liquid medicine can be injected into the user along the needle N. The user may simply rotate the needle assembly 100 to insert the needle N into the target and begin injection of the liquid medicine.

The reservoir unit 200 is mounted to the receiving portion implemented in the base body 13 and the control module 700 and is connected to the needle assembly 100. The reservoir unit 200 includes a reservoir 210, and a plunger 230 that is disposed inside the reservoir and is actuated by the pump module 300 and the drive unit 400 to move linearly. The liquid medicine may be stored in an inner space of the reservoir 210, and a set dose of the liquid medicine may move through the needle N, in response to movement of the plunger 230.

The pump module 300 may generate driving force and transmit the driving force to the drive unit 400. The pump module 300 may be any type of device that has suction force and discharge force for the liquid medicine by electricity. For example, all types of pumps, such as mechanical displacement micropumps, electromagnetic motion micropumps, and the like may be used as the pump module 300.

The drive unit 400 may be disposed between the pump module 300 and the reservoir unit 200, and move the plunger 230 disposed inside the reservoir 210 in advance using the driving force generated by the pump module 300, such that the liquid medicine is discharged through the needle N.

The pump module 300 and the drive unit 400 are operably engaged with each other by the clutch unit 500. When the drive units 400 are connected to each other by the clutch unit 500, the pump module 300 may rotate a drive wheel 420 of the drive unit 400. In response to the rotation of the drive wheel 420, a rod 410 connected to the plunger 230 may move linearly and the plunger 230 may move accordingly.

The trigger member 600 may generate a mechanical signal that causes the liquid medicine of the liquid medicine injection device 10 to be injected. The trigger member 600 may be rotatably disposed on one side of the base body 13. The trigger member 600 may rotate to initiate the operation of the pump module 300 and simultaneously the clutch unit 500 may operably connect the drive unit 400.

In detail, when the user rotates the needle assembly 100, a knob of the needle assembly 100 may apply force to an end portion of the trigger member 600, thereby initiating the rotation of the trigger member 600. When the trigger member 600 rotates, the clutch unit 500 may be activated to couple and connect the rod 410 and the drive wheel 420 of the drive unit 400, such that the plunger 230 can move forward.

The battery 350 may supply electricity to the liquid medicine injection device 1 to activate each component. In the drawings, a pair of batteries 350 are shown, but the present disclosure is not limited thereto, and the number of batteries may be set in various ways depending on the capacity, usage range, usage time, etc. of the liquid medicine injection device 1.

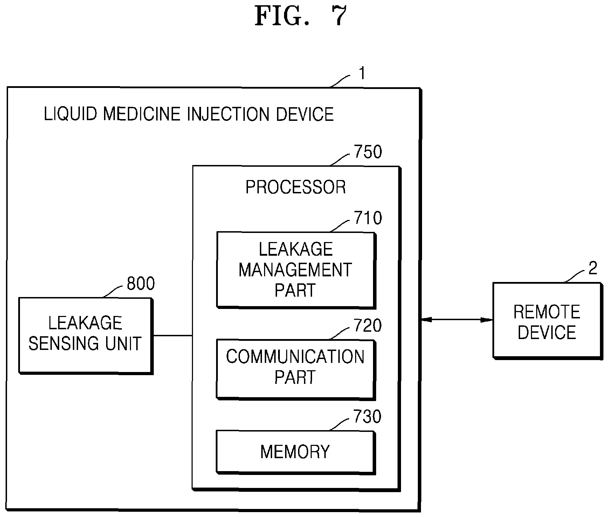

The control module 700 may be disposed below the second body 13b and may be covered by the bottom cover 17. The control module 700 is a circuit board on which at least one processor (750 in FIG. 7) is mounted. The control module 700 may have a space for accommodating the needle assembly 100, the reservoir unit 200, the pump module 300, the battery 350, etc. The control module 700 includes movement paths (not shown), for example, lines of electrical signals for electrically connecting the processor (750 in FIG. 5) to the pump module 300, the battery 350, the leakage sensing unit 800, the alarm unit 900, etc. The processor (750 in FIG. 7) may control the overall operation of the liquid medicine injection device 1. A detailed description of the processor (750 in FIG. 7) will be given later.

The leak sensing unit 800 may be mounted on the control module 700. The leakage sensing unit 800 may be connected to the line on the control module 700 to receive a voltage and transmit a signal to the processor (750 in FIG. 7). The leakage sensing unit 800 may be located anywhere on the control module 700, but may be especially located adjacent to a place where the liquid medicine is stored or adjacent to a path along which the liquid medicine moves. According to one embodiment, the leakage sensing unit 800 may be disposed at each of portions, on which liquid leakage is likely to occur, on a surface facing the external housing 11, namely, a "top surface" of the control module 700. For example, the leakage sensing unit 800 may be disposed adjacent to the reservoir unit 200 or the needle assembly 100. According to another embodiment, the leakage sensing unit 800 may be disposed at each of portions, on which liquid leakage is likely to occur, on a surface facing the bottom cover 17, namely, a "bottom surface" of the control module 700. For example, the leakage sensing unit 800 may be disposed adjacent to the reservoir unit 200 or the needle assembly 100. According to still another embodiment, the leakage sensing unit 800 may also be disposed at a portion adjacent to the base body 13, namely, a "side surface" of the control module. Meanwhile, in addition to the portions described above, the leakage sensing unit 800 may be disposed at any portion where external liquid is likely to be introduced, such as a portion adjacent to the base body 13, a portion adjacent to the battery 350 or the drive unit 400, or a top surface, bottom surface, one side surface, or both side surfaces of the control module 700 on a side corresponding to the bottom cover 17. A detailed description of the leakage sensing unit 800 will be given later.

The battery 350 may be disposed adjacent to the drive unit 400 and may supply electricity to the drive unit 400. Additionally, the battery 350 may be connected to the control module 700 and may supply a voltage to the leakage sensing unit 800 through the control module 700.

The alarm unit 900 may be disposed inside or outside the liquid medicine injection device 1 and may notify the user of normal operation or malfunction of the liquid medicine injection device 1. For example, the alarm unit 900 may notify the user of whether a leakage has occurred through an alarm. The alarm unit 900 may transmit an alarm to the user by generating warning sound, generating light, or generating vibration.

The alarm unit 900 is disposed below the external housing 11 and is electrically connected to the control module 700, which is a circuit board. A movement path (not shown) of an electrical signal, through which an alarm signal is to be transmitted to the alarm unit 900 may be formed on the control module 700.

FIG. 7 is a block diagram functionally illustrating a configuration of the processor 750 according to one exemplary embodiment of the present disclosure.

The processor 750 may be mounted on the control module 700 and may control the overall operation of the liquid medicine injection device 1. The processor 750 may be electrically connected to the pump module 300, the battery 350, the leakage sensing unit 800, the alarm unit 900, etc. to control their overall operations, and communicate with the remote device 2.

Here, the processor 750 may be implemented as a micro controller unit (MCU). However, the processor 750 is not limited thereto and may include at least one of a digital signal processor (DSP), microprocessor, time controller (TCON), central processing unit (CPU), micro processing unit (MPU), controller, application processor (AP), communication processor (CP), or ARM processor that processes digital signals, or may be defined by the corresponding term. Additionally, the processor may be implemented as a system on chip (SoC) with a built-in processing algorithm or a large-scale integration (LSI), or may be implemented in the form of a field programmable gate array (FPGA).

According to one embodiment of the present disclosure, the processor 750 may be electrically connected to the leakage sensing unit 800 to receive signals, and manage leakage issues while communicating with the remote device 2. Here, the leakage issues include not only an issue that a liquid medicine stored in the liquid medicine injection device 1 leaks out of the liquid medicine injection device 1 but also an issue that external liquid is introduced into the liquid medicine injection device 1. Hereinafter, a detailed description will be given of the contents of the processor 750 related to the leakage issues will be given.

Referring to FIG. 7, the processor 750 may include a leakage management part 710, a communication part 720, and a memory 730. However, these components are illustrative, and the control module 700 may include additional components or some of those components described above may be omitted as needed.

The leakage management part 710 analyzes an electrical signal or data input from the leakage sensing unit 800 to determine whether liquid leakage has occurred, a location where the leakage has occurred, and a type of liquid which has been leaked or introduced. Additionally, when an issue related to leakage has occurred, the leakage management part 710 may notify the user of the leakage issue by controlling the alarm unit 900 or generating a message to be sent to the remote device 2.

The communication part 720 performs data exchange and communication with the remote device 2 through the aforementioned communication network, transmits the message generated in the leakage management part 710 to the remote device 2, and receives data from the remote device 2.

The memory 730 has a function of temporarily storing data received from the leakage sensing unit 800, temporarily storing data processed by the leakage management part 710, or temporarily storing data from the remote device 2. Additionally, the memory 730 stores information and data necessary for determining a location where leakage has occurred and a type of liquid that has been leaked or introduced.

FIG. 7 illustrates that the processor 750 is included in the control module 700 of the liquid medicine injection device 1, but the present disclosure is not limited thereto. According to an optional embodiment, the processor 750 may alternatively be configured as a component included in the remote device 2.

FIG. 8 is a diagram illustrating the leakage sensing unit 800 according to one exemplary embodiment of the present disclosure. FIG. 9 is a diagram illustrating the leakage sensing unit 800 according to another exemplary embodiment of the present disclosure.

According to embodiments of the present disclosure, the leakage sensing unit 800 includes at least one leakage sensing pad 810.

The leakage sensing pad 810 is installed on the control module 700, which is the circuit board (see FIGS. 5 and 6). The leakage sensing pad 810 includes a first pattern 811 electrically connected to a power source through the control module 700, and a second pattern 812 electrically connected to the processor (750 in FIG. 7) through the control module 700. A gap 813 of a certain size is formed between one boundary of the first pattern 811 and one boundary of the second pattern 812.

For example, the first pattern 811 of the leakage sensing pad 810 may be connected to the battery through an electrical movement path on the circuit board to receive a certain reference voltage V_{DD}. The second pattern 812 may be connected to an input pin of the processor (750 in FIG. 7) through the electrical movement path on the circuit board to input a predetermined signal to the leakage management part (710 in FIG. 7). Here, the signal may be a digital signal or an analog signal.

The leakage sensing unit 800 may further include a voltage divider 820. The voltage divider 820 may be, for example, a resistor, and may be electrically connected between one end of the second pattern 812, which is electrically connected to the processor (750 in FIG. 7), and the input pin of the processor (750 in FIG. 7), to ensure a stable operation of the leakage sensing unit 800.

The voltage divider 820 is selected to have a resistance value greater than resistance of liquid that has been leaked or introduced. This is because if resistance smaller than the resistance of the leaked liquid is selected, current consumption increases, making it difficult to actuate the leakage sensing unit 800 stably. For example, if the liquid medicine injection device is for injecting insulin, resistance of the insulin is about 300 kΩ (kilohm) to 500 kΩ. Therefore, in this case, the resistance value of the voltage divider may be selected to be about 800 to 1200 kΩ (kilohm), preferably, 1 MΩ (megaohm).

FIG. 8 illustrates an embodiment in which a plurality of leakage sensing pads 810 are electrically connected to one input pin to transmit a comprehensive signal (sensing) to the processor (750 in FIG. 7). In this embodiment, the processor (750 in FIG. 7) may merely determine whether a leakage has occurred from the liquid medicine injection device 1.

In FIG. 9, a plurality of leakage sensing pads 810 may be electrically connected to a plurality of input pins, respectively, to transmit respective signals sensing_1 to sensing_N to the processor (750 in FIG. 7). In this embodiment, the processor (750 in FIG. 7) may determine "at what location" within the liquid medicine injection device 1 leakage has occurred, in addition to whether the leakage occurred.

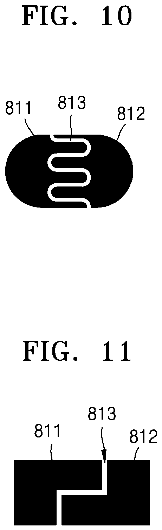

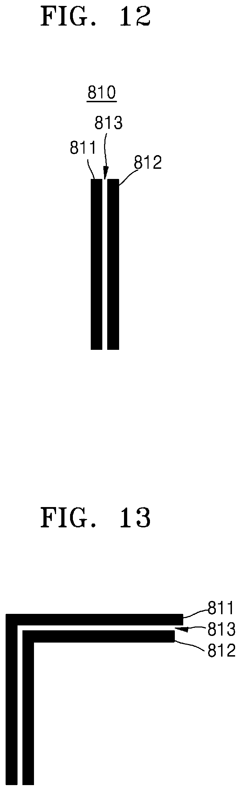

FIGS. 10 to 13 are diagrams illustrating various types of leakage sensing pads.

As illustrated in FIGS. 10 to 13, the leakage sensing pad 810 may be implemented in various shapes. For example, the leakage sensing pad 810 may be implemented in a circular, polygonal, linear, broken-line, or curved shape, and the shape is not limited to what is illustrated and what is described above, and may be formed in any shape if the certain gap 813 is formed between the first pattern 811 and the second pattern 812.

Additionally, in order to facilitate leakage sensing and increase a sensing effect, the boundary of the first pattern 811 and the adjacent boundary of the second pattern 812 may be designed to have surface areas adjacent to each other as wide as possible. For example, the gap 813 between the first pattern 811 and the second pattern 812 may be designed to have a curved or zigzag shape to increase a rate of success for leakage sensing.

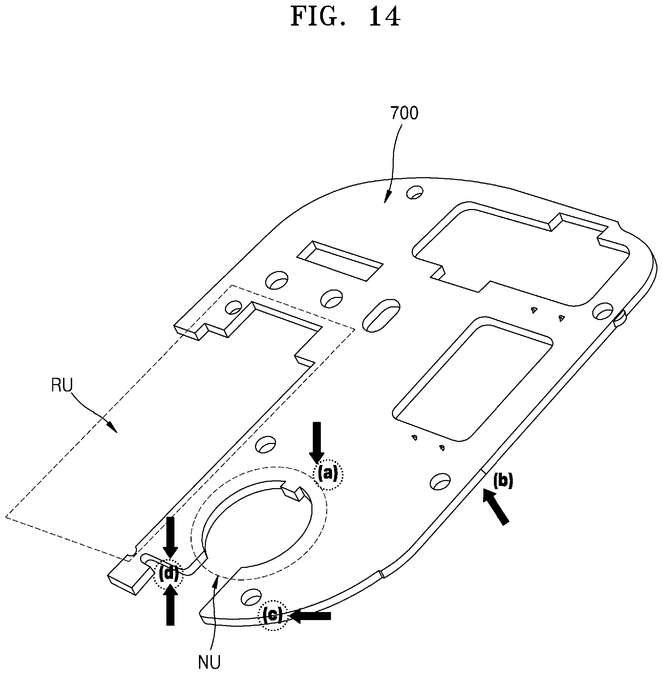

FIG. 14 is a schematic view illustrating positions of the leakage sensing pads 810 on the control module 700.

As illustrated in FIG. 14, the leakage sensing pad 810 is disposed on any portion where leakage is likely to occur, for example, on a top (a), bottom (b), side surface (c), or both side surfaces (d) of the control module 700, without limits. Here, in the case of disposition on the both side surfaces (d), one pattern may be disposed on a top surface while the other pattern may be disposed on a bottom surface, and a gap corresponding to a thickness of the control module may be formed between the both patterns. As described above, the leakage sensing pad 810 may be located on a circuit board adjacent to the reservoir unit (RU location) where the liquid medicine is stored or adjacent to the needle assembly (NU location) through which the liquid medicine moves.

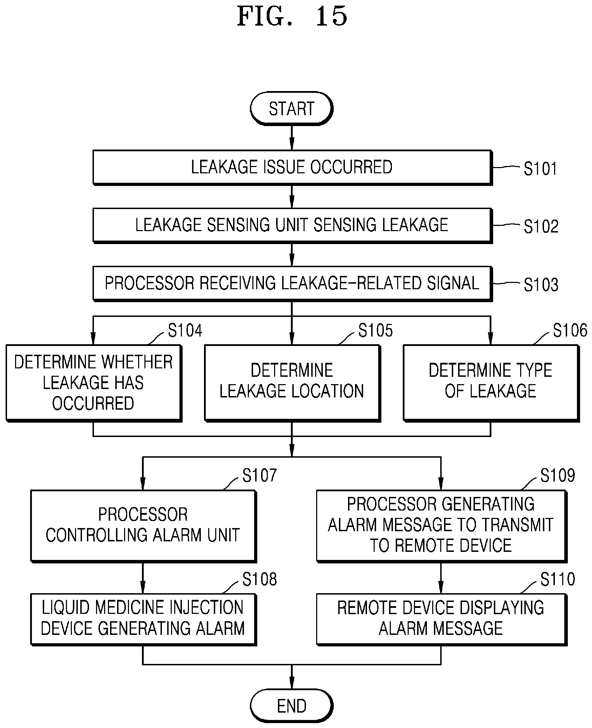

FIG. 15 is a flowchart for a leakage management method of the liquid medicine injection system S or the liquid medicine injection device 1.

Referring to FIG. 15, a leakage issue occurs in step 101.

The liquid medicine injection device (1 in FIG. 1) may be attached to the user with the liquid medicine stored therein and may inject the liquid medicine into the user by a set dosage. The liquid medicine is stored in the inner space of the reservoir unit (200 in FIG. 2), and the liquid medicine is injected into the user through the needle (N in FIG. 2). However, the liquid medicine may be leaked due to unexpected circumstances in the process of storing the liquid medicine or attaching the liquid medicine injection device 1 for use. Additionally, when exposed to an environment exceeding a designed waterproofing specification, a liquid may be introduced into the liquid medicine injection device 1 from outside. In order to ensure stability and reliability of the liquid medicine injection device 1, whether a leakage has occurred must be sensed and notified to the user before the control module (700 in FIG. 5), which is the circuit board, is damaged or the liquid medicine injection device 1 is turned off due to a leaked liquid medicine or introduced liquid.

In step 102, the leakage sensing unit (800 in FIGS. 5 and 8) senses a leakage.

The leakage sensing pad 810 included in the leakage sensing unit 800 includes a first pattern 811 and a second pattern 812 made of a conductive material. A reference voltage V_{DD} may be applied to the first pattern 811 and the second pattern 812 may be connected to ground or a low voltage through the voltage divider 820. When no leakage issue has occurred, the first pattern 811 and the second pattern 812 maintain a certain gap 813 therebetween without being in contact with each other. Accordingly, the leakage management part (710 in FIG. 7) electrically connected to the second pattern 812 confirms a voltage value of 0V or that there is no input of a voltage value. However, when a leakage issue has occurred, a liquid intervenes between the first pattern 811 and the second pattern 812, creating resistance by the liquid between both the conductive patterns. Due to the resistance, a voltage value which has changed is input to the processor (750 in FIG. 7) which is electrically connected to the second pattern 812.

In step 103, the processor (750 in FIG. 7) receives a leakage-related signal from the leakage sensing unit 800.

When the leakage issue has occurred, the processor (750 in FIG. 7) receives a leakage signal, such as the changed voltage value from the leakage sensing unit 800. In an optional embodiment, the leakage management part (710 in FIG. 7) may alternatively receive an on/off signal or a high/low signal as a leakage signal.

In step 104, the processor (750 in FIG. 7) may determine whether a leakage has occurred.

The leakage management part (710 in FIG. 7) of the processor (750 in FIG. 7) may determine whether a leakage has occurred by analyzing the leakage signal input from the leakage sensing unit 800. For example, the leakage management part (710 in FIG. 7) may determine that a leakage issue has occurred when the change of the voltage value input from the leakage sensing unit 800 reaches a set range. In an optional embodiment, when receiving an on/off signal or a high/low signal as a leakage signal, the leakage management part (710 in FIG. 7) may determine that a leakage issue has occurred through the change of the signal.

In step 105, as an optional embodiment, the processor (750 in FIG. 7) may determine a location where a leakage has occurred.

In the case of the leakage sensing unit 800 shown in FIG. 9, each leakage sensing pad 810 may input an individual leakage signal to the processor (750 in FIG. 7). Accordingly, the leakage management part (710 in FIG. 7) of the processor (750 in FIG. 7) may determine the location where the leakage has occurred by confirming from which leakage sensing pad the leakage signal has been input. Information linking a leakage signal and a leakage occurrence location may be stored in the memory (730 in FIG. 7) of the processor (750 in FIG. 7), and the leakage management part (710 in FIG. 7) may determine the leakage occurrence location using the information stored in the memory (730 in FIG. 7).

In step 106, as an optional embodiment, the processor (750 of FIG. 7) may determine a type of leakage (i.e., a type of liquid that has been leaked).

The leakage sensing units 800 illustrated in FIGS. 8 and 9 may input voltage values, respectively, as leakage signals, to the processor (750 in FIG. 7). The leakage management part (710 in FIG. 7) of the processor (750 in FIG. 7) may determine which liquid has caused the leakage issue when the change of a voltage value input from the leakage sensing unit 800 reaches a specific range. For example, resistance of insulin is in the range of about 300 kΩ (kilohm) to 500 kΩ (kilohm). In the case where a reference voltage V_{DD} input to one end of the leakage sensing unit 800 is 3 V, when insulin is sensed on the leakage sensing pad 810, a voltage of about 2 V to 2.3 V is input as a leakage signal to the leakage management part (710 in FIG. 7). Therefore, when the voltage of about 2 V to 2.3 V is input as the leakage signal, the liquid medicine injection device 1 may determine that insulin has been leaked or introduced. For example, resistance of water is in the range of about 2 kΩ (kilohm) to 200 kΩ (kilohm). In the case where a reference voltage V_{DD} input to one end of the leakage sensing unit is 3 V, when water is sensed on the leakage sensing pad 810, a voltage of about 2.5 V to 2.9 V is input as a leakage signal to the leakage management part (710 in FIG. 7). Therefore, when the voltage of about 2.5 V to 2.9 V is input as the leakage signal, the liquid medicine injection device 1 may determine that water has been leaked or introduced. For example, resistance of salt water is about 0.2 Ω (ohm). In the case where a reference voltage V_{DD} input to one end of the leakage sensing unit 800 is 3 V, when salt water is sensed on the leakage sensing pad 810, a voltage of about 3 V is input as a leakage signal to the leakage management part (710 in FIG. 7). Therefore, when the voltage of about 3 V is input as the leakage signal, the liquid medicine injection device 1 may determine that salt water has been leaked or introduced. Information linking the leakage signal and a type of leakage may be stored in the memory (730 of FIG. 7) of the processor (750 of FIG. 7). Accordingly, the leakage management part (710 in FIG. 7) may use the information stored in the memory (730 in FIG. 7) to determine which liquid has been leaked or introduced based on the leakage signal, specifically, the voltage value.

Since the leakage issue has been confirmed, the leakage management part (710 in FIG. 7) notifies the user of the leakage issue in various ways.

In step 107, the processor (750 in FIG. 7) may generate a leakage alarm by controlling the alarm unit 900 electrically connected to the control module 700.

In step 108, the alarm unit 900 may notify the user of the leakage issue by generating at least one of light or vibration. For example, the alarm unit 900 may notify the user of the leakage issue by generating sound, light, or vibration at predetermined intervals, generating sound of a specific pitch, intensity, or style, generating light of a specific color or interval, or generating light of specific intensity, interval, or time.

In an optional embodiment, in step 109, the leakage management part (710 in FIG. 7) may generate an alarm message notifying that an issue related to leakage has occurred and control the communication part (720 in FIG. 7) to transmit the alarm message to the remote device 2. The alarm message may include information related to the leakage occurrence location determined in step S105, and the type of leakage determined in step S106, in addition to information about whether the leakage has occurred.

In step 110, the remote device 2 connected to the liquid medicine injection device 1 through the communication network receives the alarm message and notifies the user that the leakage has occurred in the liquid medicine injection device 1 in various ways.

For example, the remote device 2 may allow the user to confirm the leakage issue by displaying the alarm message on a display screen. Through the message, the user can confirm not only whether the leakage has occurred, but also the leakage occurrence location and the type of leakage. In addition, the remote device 2 may notify the user that it has received the message related to the leakage issue through sound, vibration, light, etc.

Those steps 107, 108, 109, and 110 of the present disclosure may be performed simultaneously, or only the steps 107 and 108 or only the steps 109 and 110 may be performed depending on the user's selection or a set method.

According to an embodiment of the present disclosure, when a leakage has occurred in the liquid medicine injection device 1, the leakage can be sensed and recognized by the user, which provides effects of suppressing in advance damage to the circuit board and forced turn-off of the system due to the leakage and enhancing reliability of the device.

According to an embodiment of the present disclosure, when a leakage has occurred in the liquid medicine injection device 1, it is possible to determine not only whether the leakage has occurred, but also the location where a liquid has been leaked or introduced or a type of liquid that has been leaked or introduced. This allows the user to respond to the leakage issue quickly and accurately.

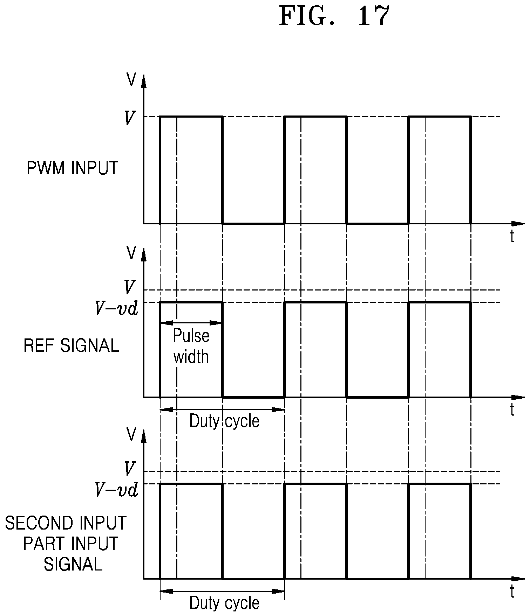

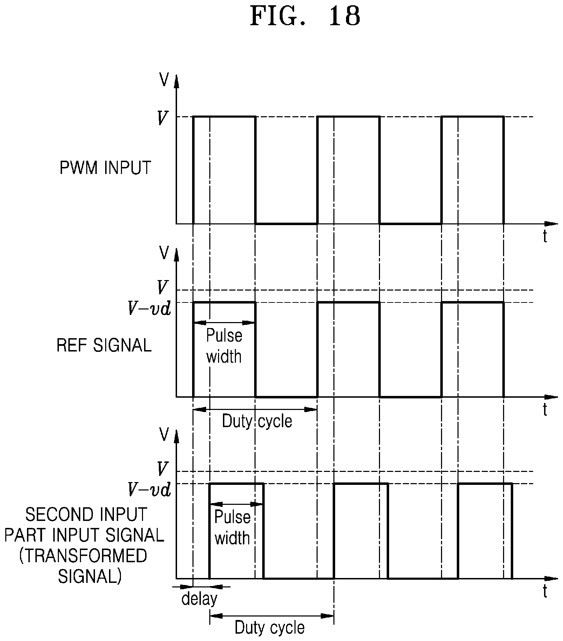

FIG. 16 is a diagram illustrating the leakage sensing unit 800 according to another exemplary embodiment of the present disclosure. FIGS. 17 and 18 are diagrams illustrating signals related to the leakage sensing unit 800 of FIG. 16.

According to another embodiment of the present disclosure, the leakage sensing unit 800 further includes a comparator 840 in addition to the leakage sensing pad 810.

Since the leakage sensing pad 810 has been described in detail previously, the following description will be given of the comparator 840 and the operation of the leakage sensing unit 800 including the same, and redundant descriptions will be omitted.

The comparator 840 includes a first input part ① and a second input part ②, and the first input part ① and the second input part ② are electrically connected to a waveform generator 850 to receive predetermined waveforms. One end of the leakage sensing pad 810, for example, the first pattern 811 may be electrically connected between the waveform generator 850 and the second input part ②. The other end of the leakage sensing pad 810, for example, the second pattern 812 may be electrically connected to ground or a low voltage. In the leakage sensing pad 810, the first pattern 811 and the second pattern 812 made of the conductive material have a predetermined gap therebetween without being in contact with each other. In a general case where no issue occurs, a reference signal is input to the first input part ① of the comparator 840, and the same reference signal is also input to the second input part ②. Here, a signal generated from the waveform generator 850, as illustrated in FIGS. 17 and 18, may be a pulse width modulation (PWM) signal, but is not limited thereto, and may alternatively be a square wave or a sine wave.

According to one embodiment, an output value of the comparator 840 may have a first value or a second value, and is input to the processor (750 in FIG. 7). When the same signal is input to the first input part ① and the second input part ② of the comparator 840, the comparator may output the first value (for example, a low value). On the other hand, when different signals are input to the first input part ① and the second input part ②, the comparator may output the second value (for example, a high value). In this case, the leakage management part (710 in FIG. 7) of the processor (750 in FIG. 7) may determine that a leakage issue has not occurred when the first value is input, and may determine that a leakage issue has occurred when the second value is input.

Meanwhile, in an optional embodiment, diodes 860 for protecting elements from reverse current may be interposed between the waveform generator 850 and the first input part ① and between the waveform generator 850 and one end of the leakage sensing pad 810.

As illustrated in FIG. 17, when no leakage issue has occurred, the first pattern 811 and the second pattern 812 maintain a predetermined gap without being in contact with each other. Therefore, the signal input to the second input part ② of the comparator 840 is not affected by the leakage sensing pad 810. A signal (second input part input signal) input to the second input part ② of the comparator 840 has the same duty and pulse width as a reference signal (Ref signal) and has no delay. Therefore, the comparator 840 outputs the first value, and this output value is input to the processor (750 in FIG. 7). The leakage management part (710 in FIG. 7) determines that no leakage issue has occurred.

As illustrated in FIG. 18, when a leakage issue has occurred, a liquid intervenes between the first pattern 811 and the second pattern 812 to thereby generate resistance between both the conductive patterns. Accordingly, the signal input to the second input part ② is transformed due to capacitance of the leakage sensing pad 810. For example, the signal input to the second input part ② (second input part input signal) may be a signal that has a different duty from the reference signal (Ref signal), has a different pulse width from the reference signal, or has a delay compared to the reference signal. Therefore, the comparator 840 outputs the first value, and this output value is input to the processor (750 in FIG. 7). The leakage management part (710 in FIG. 7) determines that a leakage issue has occurred.

In an optional embodiment, the leakage management part (710 in FIG. 7) may determine a type of leakage based on the output value of the comparator 840. As described above, since each liquid has different resistance, the second input value ② input to the comparator 840 may generate a transformed signal which has an inherent duty, pulse width, and/or delay depending on the type of each liquid leaked or introduced. Therefore, when a leakage issue has occurred, the comparator 840 may output the output value of the comparator as a signal having a certain duty, pulse width, and/or delay by comparing the reference signal input to the first input part ① and the transformed signal input to the second input part ②. The output value of the comparator 840 is input to the processor (750 in FIG. 7). Information linking the output signal and the type of liquid leaked or introduced (type of leakage) may be stored in the memory (730 of FIG. 7) of the processor (750 of FIG. 7). Accordingly, the leakage management part 710 may determine which liquid has been leaked or introduced by analyzing the output value of the leakage sensing unit 800 based on the information stored in the memory 730.

The embodiment according to the present disclosure described above may be implemented as a computer program that can be executed through various components on a computer, and such a computer program may be recorded in a computer-readable medium. At this time, examples of such media may include magnetic media such as hard disk, floppy disk, and magnetic tape, optical recording media such as CD-ROM and DVD, magneto-optical media such as floptical disk, and hardware devices such as ROM, RAM, flash memory, etc. which are specifically configured to store and execute program instructions.

Meanwhile, the computer program may be designed and configured especially for the present disclosure, or may be known to those skilled in the art of computer software for use. Examples of such computer programs may include not only machine language codes created by a compiler, for example, but also high-level language codes executable by a computer using an interpreter or the like.

The specific implementations described in the present disclosure are merely illustrative, and do not limit the range of the present disclosure in any way. For the sake of brevity of the specification, descriptions of related art electronic components, control systems, software, and other functional aspects of the systems may be omitted. In addition, connections or connection members of lines between components illustrated in the drawings exemplify functional connections and/or physical or circuit connections, and in actual devices, may be replaceable or may be represented as additional various functional connections, physical connections, or circuit connections. Furthermore, unless otherwise indicated obviously by terms, such as "essential," "important," etc., a component may not be a necessary component for the application of the present disclosure.

In the specification (particularly, in the claims) of the present disclosure, the use of the term "the" and similar referential terms may refer to both the singular and the plural. In addition, when a range is described in the present disclosure, it includes an invention to which individual values belonging to the range are applied (unless otherwise described contrarily), which means that each individual value constituting the range is described in the detailed description of the invention. Finally, unless an order of steps constituting a method according to the present disclosure is explicitly described or a reverse order is not mentioned, those steps may be carried out in any suitable order. The present disclosure is not necessarily limited by the order of steps described. The use of any examples or illustrative terms (e.g., etc.) in the present disclosure is merely to describe the present disclosure in detail, and unless limited by the claims, the range of the present disclosure is not limited by the examples or illustrative terms. Additionally, it will be understood by those skilled in the art that various modifications, combinations and changes may be made depending on design conditions and factors within the range of the appended claims or their equivalents. As such, the present disclosure has been described with reference to an embodiment shown in the drawings, but is merely illustrative, and it will be understood by those of skilled in the art that various modifications and variations of the embodiment can be made from the embodiment. Therefore, the true technical protection scope of the present disclosure should be defined by the technical ideas of the appended claims.

### Industrial Applicability

One embodiment of the present disclosure relates to a liquid medicine injection device that senses leakage of liquid medicine or introduction of external liquid.

## Claims

1. A liquid medicine injection device that is attached to a user's body to inject a liquid medicine, the liquid medicine injection device comprising:
a leakage sensing unit that is mounted on a control module and comprising at least one leakage sensing pad for generating a leakage signal when sensing a liquid; and
a processor that is mounted on the control module and electrically connected to the leakage sensing unit, to determine whether a leakage has occurred by receiving the leakage signal and notify the user of information related to the leakage when the leakage has occurred.

2. The liquid medicine injection device of claim 1, further comprising:
an alarm unit that is electrically connected to the processor to notify the user of the information related to the leakage by generating an alarm of sound, light, or vibration; and
a remote device that notifies the user of the information related to the leakage through communication with the processor.

3. The liquid medicine injection device of claim 1, wherein
the information related to the leakage includes at least one of whether the leakage has occurred, a location where the leakage has occurred, and a type of leakage that has occurred.

4. The liquid medicine injection device of claim 1, wherein
the at least one leakage sensing pad each comprises a first pattern electrically connected to a power source, and a second pattern electrically connected to the processor and spaced a certain gap from the first pattern, and
the leakage sensing unit further comprises:
the at least one leakage sensing pad connected in parallel; and
a voltage divider interposed between the at least one leakage sensing pad connected in parallel and the processor.

5. The liquid medicine injection device of claim 1, wherein
the leakage sensing unit comprises:
the at least one leakage sensing pad each having a first pattern electrically connected to a power source, and a second pattern electrically connected to the processor and spaced a certain gap from the first pattern; and
at least one voltage divider interposed between the second pattern of each of the at least one leakage sensing pad and the processor, and
the at least one leakage sensing pad each is mounted on the control module at a different position, and the leakage sensing unit generates the leakage signal from the at least one leakage sensing pad and inputs the same to the processor.

6. The liquid medicine injection device of claim 4, wherein
the leakage signal is a voltage value, and the processor determines a type of leakage based on the voltage value and notifies the user of the determined type of leakage as the information related to the leakage.

7. The liquid medicine injection device of claim 1, wherein
the leakage sensing unit comprises
a comparator that receives a reference signal through a first input part, receives a transformed signal from a second input part, and outputs the leakage signal, and
the at least one leakage sensing pad is electrically connected to the second input part, to input the transformed signal to the second input part when the leakage has occurred.

8. The liquid medicine injection device of claim 7, wherein
the leakage signal has a waveform, and the processor determines a type of leakage based on at least one of a duty, pulse width, and delay of the waveform, and notifies the user of the determined type of leakage as the information related to the leakage.

9. The liquid medicine injection device of claim 1, wherein
the liquid medicine injection device further comprises:
a needle assembly that is mounted on a first receiving portion formed in the control module and has a needle; and
a reservoir unit that is mounted on a second receiving portion formed in the control module, accommodates the liquid medicine, and is connected to one end of the needle to deliver the accommodated liquid medicine to the user.

10. The liquid medicine injection device of claim 9, wherein
the at least one leakage sensing pad is located on the control module to be adjacent to the reservoir unit and to the needle assembly.
